(19) Europäisches Patentamt
European Patent Office
Office européen des brevets

(11) Veröffentlichungsnummer: **0 529 226 B1**

(12) # EUROPÄISCHE PATENTSCHRIFT

(45) Veröffentlichungstag der Patentschrift: **28.12.94**

(51) Int. Cl.[5]: **C07C 65/24**, C07C 69/92, A61K 31/19, A61K 31/235

(21) Anmeldenummer: **92110602.7**

(22) Anmeldetag: **24.06.92**

(54) **Neue p-Oxibenzoesäurederivate, Verfahren zu ihrer Herstellung und ihre Verwendung als Arzneimittel.**

(30) Priorität: **22.08.91 DE 4127800**

(43) Veröffentlichungstag der Anmeldung:
**03.03.93 Patentblatt 93/09**

(45) Bekanntmachung des Hinweises auf die Patenterteilung:
**28.12.94 Patentblatt 94/52**

(84) Benannte Vertragsstaaten:
**AT BE CH DE DK ES FR GB GR IT LI LU MC NL PT SE**

(56) Entgegenhaltungen:
**EP-A- 0 133 935**
**DE-A- 2 460 689**

(73) Patentinhaber: **Klinge Pharma GmbH**
**Berg-am-Laim-Strasse 129**
**D-81673 München (DE)**

(72) Erfinder: **Reiter, Fiedemann, Dr. Dipl.-Chem.**
**Zugspitzstrasse 36**
**W-8011 Putzbrunn (DE)**
Erfinder: **Grill, Helmut, Dr. Dipl.-Chem.**
**Zugspitzstrasse 146**
**W-8011 Vaterstetten (DE)**
Erfinder: **Henschel, Hans-Helmut**
**Gudrunstrasse 18**
**W-8000 München 19 (DE)**
Erfinder: **Schliack, Michael**
**Neumarkter Strasse 82**
**W-8000 München 80 (DE)**
Erfinder: **Seibel, Klaus, Prof. Dr.**
**Haberlstrasse 9**
**W-8032 Gräfelfing (DE)**
Erfinder: **Löser, Roland, Dr.**
**Fichtenweg 2**
**W-8133 Feldafing (DE)**
Erfinder: **Lang, Gerhard**
**Landshuter Strasse 114**
**W-8050 Freising (DE)**

(74) Vertreter: **von Hellfeld, Axel, Dr. et al**
**Schweigerstrasse 2**
**D-81541 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Aus der DE-OS 33 26 164 sowie der EP-A-0-133 935 sind therapeutisch wertvolle p-Oxibenzoesäurederivate der allgemeinen Formel:

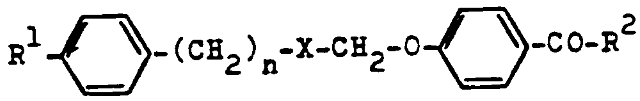

in der bedeuten:

$R^1$ =

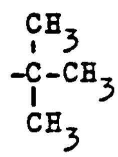 oder 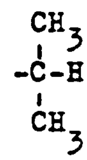

n = 1 oder 2

X =

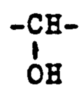 oder 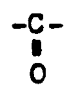

und

$R^2$ = -OH oder $-NHCH_2COOH$

ihre physiologisch verträglichen Salze, Verfahren zu ihrer Herstellung sowie ihre Verwendung zur Herstellung von Arzneimitteln mit hypolipämischer Wirkung bekannt.

In der deutschen Patentanmeldung P 40 32 037.5 werden ferner p-Oxibenzoesäurederivate mit hypolipämischen Eigenschaften der folgenden Formel:

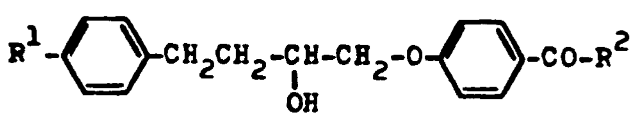

in der bedeuten:

$R^1$ =

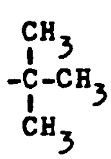 , 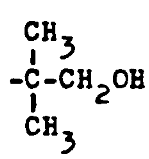 oder 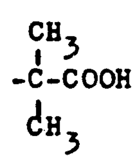

und

$R^2$ = -OH ,

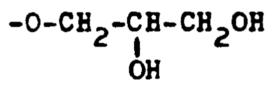 oder 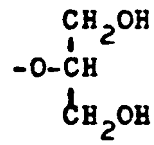 )

wobei im Falle von

$R^1$ =

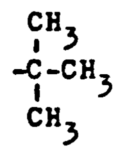

$R^2$ =

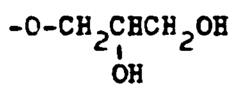 oder 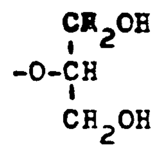

bedeutet

und im Falle von

$R^1$ =

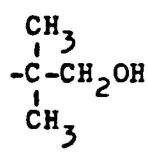 oder 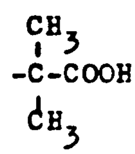

$R^2$ = -OH bedeutet, ihre physiologisch verträglichen Salze, Verfahren zu ihrer Herstellung sowie ihre Verwendung zur Herstellung von Arzneimitteln beschrieben.

Überraschenderweise wurde nun gefunden, daß bezüglich ihrer hypolipämischen Wirkung besonders vorteilhafte p-Oxibenzoesäurederivate solche sind, die sich von den aus der DE-OS 33 26 164 und der EP-A-O 133 935 bekannten sowie von den in der deutschen Patentanmeldung P 40 32 037.5 beschriebenen p-Oxibenzoesäurederivaten im wesentlichen dadurch unterscheiden, daß die die beiden Arylreste verbindende aliphatische Kette entsprechend dem folgenden Formelbild zwei Hydroxygruppen aufweist:

(1) 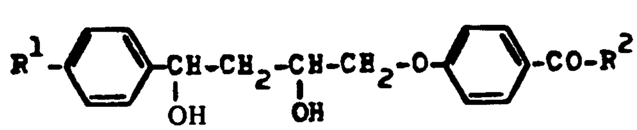

Gegenstand der Erfindung sind demzufolge p-Oxibenzoesäurederivate der allgemeinen Formel (1), in der bedeuten:

$R^1$ =

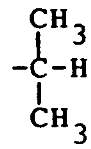 , 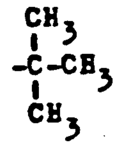 , 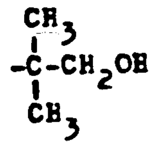 oder 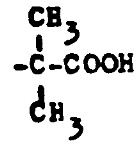

und

$R^2$ = -OH ,

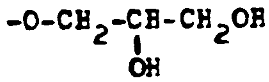 oder 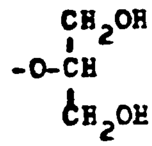 ,

ihre Diastereomere und Enantiomere in reiner Form oder als Gemisch stereoisomerer Formen sowie ihre physiologisch verträglichen Salze.

Unter die allgemeine Formel (1) fallen beispielsweise folgende p-Oxibenzoesäurederivate mit besonders vorteilhaften hypolipämischen Eigenschaften:

1.) 1-(4'-tert.Butylphenyl)-4-(4'-carboxyphenoxy)-butandiol-1,3;
2.) 1-(4'-Isopropylphenyl)-4-(4'-carboxyphenoxy)-butandiol-1,3;
3.) 1-[4'-(1,1-Dimethyl-2-hydroxyethyl)-phenyl]-4-(4'-carboxyphenoxy)-butandiol-1,3;
4.) 1-[4'-(1-Carboxy-1-methylethyl)-phenyl]-4-(4'-carboxyphenoxy)-butandiol-1,3;
5.) 1-(4'-tert.Butyiphenyl)-4-[4'-(2,3-dihydroxypropoxycarbonyl)-phenoxy]-butandiol-1,3;
6.) 1-[4'-(1,1-Dimethyl-2-hydroxyethyl)-phenyl]-4-[4'-(2,3-dihydroxypropoxycarbonyl)-phenoxy]-butandiol-1,3.

Die Herstellung der erfindungsgemäßen p-Oxibenzoesäurederivate kann dadurch erfolgen, daß man in an sich bekannter Weise, Epoxide der allgemeinen Formel (2)

(2) 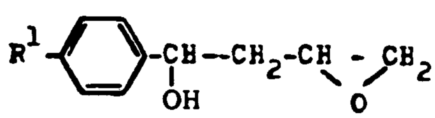

in der

$R^1$ =

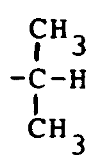 , 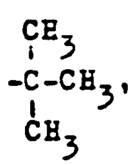 , 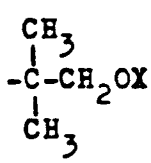 oder 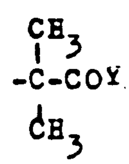

bedeutet und sowohl X als auch Y eine geeignete Schutzgruppe darstellt, mit den entsprechenden p-Oxibenzoesäureestern umsetzt, gegebenenfalls die erhaltenen Ester verseift, gegebenenfalls vorhandene Schutzgruppen abspaltet sowie gegebenenfalls das erhaltene Diastereomerengemisch trennt, wobei die Trennung vor oder nach Abspaltung der Schutzgruppe erfolgen kann.

Die Trennung der Diastereomeren kann dabei nach üblichen Methoden erfolgen, in vorteilhafter Weise durch fraktionierte Kristallisation oder auf chromatographischem Wege.

X kann beispielsweise eine Tetrahydropyranylgruppe sein und Y eine Tetrahydropyranyloxygruppe oder eine Alkoxygruppe, insbesondere kurzkettige Alkoxygruppe, wie z.B. eine Methoxygruppe.

Die Umsetzung der Epoxide mit den p-Oxibenzoesäureestern kann in einem Lösungsmittel, z.B. Dimethylformamid, in Gegenwart von Alkali, beispielsweise in Form des Alkalisalzes des eingesetzten p-Oxibenzoesäureesters, bei erhöhter Temperatur, z.B. 80 bis 130 °C, erfolgen.

Die zur Durchführung des Verfahrens der Erfindung benötigten Epoxide der allgemeinen Formel (2) lassen sich dadurch erhalten, daß man einen Aldehyd der allgemeinen Formel (3)

(3) 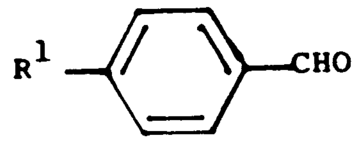

in der $R^1$ die angegebene Bedeutung hat, mit einem Allylmagnesiumhalogenid, z.B. Allylmagnesiumbromid, zu einem Phenyl-buten 3-ol-1 der folgenden Formel (4)

(4) 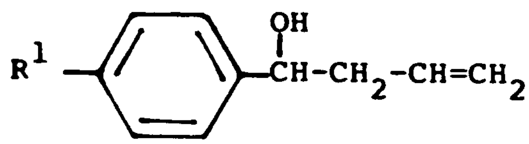

umsetzt, worauf man die erhaltene Verbindung in an sich bekannter Weise durch Oxidation, beispielsweise mittels einer organischen Persäure, z.B. meta-Chlorperbenzoesäure (MCPBA), in das Epoxid der folgenden Formel (5) überführt:

(5) 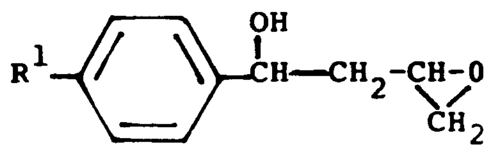

Das Epoxid fällt in Form des Diastereomerengemisches mit 2 Enantiomerenpaaren an, das man dann in der angegebenen Weise mit einem entsprechenden p-Oxibenzoesäureester umsetzt.

Beispielsweise erfolgt die Herstellung von 1-(4'-tert.Butylphenyl)-4(4'-carboxyphenoxy)-butandiol-1,3, dadurch, daß man (1) 4-tert.Butylbenzaldehyd mit einem Allylmagnesiumhalogenid, z.B. Allylmagnesiumbromid, zu 1-(4'-tert.Butylphenyl)buten-3-ol-1 umsetzt, daß man (2) das erhaltene 1-(4'-tert.Butylphenyl)-buten-3-ol-1 zu 1-(4'-tert.Butylphenyl)-3,4-epoxy-butanol-1 oxidiert, daß man (3) das erhaltene 1-(4'-tert-Butylphenyl)-3,4-epoxy-butanol-1 mit 4-Hydroxybenzoesäuremethylester zu einem 1-(4'-tert.Butylphenyl)-4-(4'-methoxycarbonylphenoxy)-butandiol-1,3-Diastereomerengemisch umsetzt, daß man (4) die Diastereomeren von einander trennt und daß man (5) die getrennten Ester alkalisch verseift.

Für die therapeutische Anwendung als hypolipämische Arzneimittel werden die neuen Verbindungen der allgemeinen Formel (1) und ihre Salze bevorzugt oral verabreicht. Gewöhnlich beträgt die orale Tagesdosis bei Erwachsenen 0,1 bis 5 g, vorzugsweise 0,3 bis 2 g.

Die Wirkstoffe können zur oralen Verabreichung in üblicher Weise galenisch verarbeitet werden. Als pharmazeutische Trägerstoffe eignen sich gängige Hilfsstoffe wie Lactose, Saccharose, Mannit, Kartoffel- oder Maisstärke, Cellulosederivate oder Gelatine, gegebenenfalls unter Zusatz von Gleitmitteln, wie z.B. Magnesium- oder Calciumstearat, sowie Polyethylenglykole.

Die Erfindung soll am Beispiel der Herstellung von 1-(4'-tert.-Butylphenyl)-4-(4'-carboxyphenoxy)-butandiol-1,3 und dessen pharmakodynamischer Effekte näher erläutert werden.

1-(4'-tert.Butylphenyl)-buten-3-ol-1

Zur Lösung von 123 ml (0.123 Mol) Allylmagnesiumbromidlösung (1 M in Diethylether) tropft man unter Stickstoffatmosphäre bei 10 °C die Lösung von 16.2 g (0.100 Mol) 4-tert.Butylbenzaldehyd in 50 ml abs.

Ether, rührt 2 Stunden bei 10 - 15 °C weiter und läßt über Nacht bei Raumtemp. stehen. Dann rührt man unter Kühlung 50 ml 25 % $NH_4Cl$-Lösung ein, trennt die Phasen und extrahiert die wässrige mehrmals mit Methyl-tert.butylether. Die vereinigten organischen Phasen werden mit Wasser gewaschen und über $Na_2SO_4$ getrocknet. Nach Abziehen des Lösungsmittels i.Vak. bleibt eine gelbliche Flüssigkeit, die ohne weitere Reinigung umgesetzt wird. Ausbeute 19.5 g (96 %).

| $^1$H-NMR-Spektrum (60 MHz; $CDCl_3$): | | | | |
|---|---|---|---|---|
| 1.33 | | s | (9) | $(C\underline{H}_3)_3C$ |
| 2.23 | breites | s | (1) | O$\underline{H}$ (austauschbar) |
| 2.30 - 2.60 | | m | (2) | $CHC\underline{H}_2CH$ |
| 4.48 - 4.83 | | t | (1) | ArC$\underline{H}$ |
| 4.83 - 5.30 | | m | (2) | $=C\underline{H}_2$ |
| 5.47 - 6.30 | | m | (1) | $=C\underline{H}$ |
| 7.10 - 7.50 | | m | (4) | Aromat |

Die NMR-Spektren sind aufgenommen bei 60 oder 100 MHz.
Die chemischen Verschiebungen sind in ppm gegen TMS ($\delta = 0.0$) angegeben, die relativen. Intensitäten sind in Klammern beigefügt. s = Singulett; d = Dublett; dd = Doppeldublett; t = Triplett; q = Quartett; m = Multiplett.

1-(4'-tert.Butylphenyl)-3,4-epoxy-butanol-1 (Diastereomerengemisch)

Zur Lösung von 19.0 g (0.093 Mol) 1-(4'-tert.Butylphenyl)-buten-3-ol-1 in 100 ml Dichlormethan gibt man unter Eiskühlung 18.9 g (0.093 Mol) 85 % MCPBA und rührt anschließend 5 Stunden bei Raumtemp. Danach wird die suspendierte Chlorbenzoesäure abgesaugt, das Filtrat mit verd. NaOH säurefrei und mit Wasser neutral gewaschen und über $Na_2SO_4$ getrocknet. Nach Abziehen des Lösungsmittels i. Vak. bleibt ein gelbes Öl (21 g), das chromatographisch mit Chloroform an Kieselgel gereinigt wird. Ausbeute 13 g (59 %).

**$^1$H-NMR-Spektrum (60 MHz; $CDCl_3$):**

| | | | | |
|---|---|---|---|---|
| 1.28 | | s | (9) | $(C\underline{H}_3)_3$ |
| 1.47 - 2.13 | | m | (2) | $CHC\underline{H}_2CH$ |
| 2.25 - 3.30 | | m | (4) | 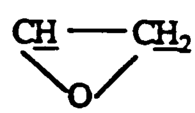 |
| 2.97 | breites | s | | O$\underline{H}$ |
| 4.57 - 5.02 | | m | (1) | ArC$\underline{H}$ |
| 7.00 - 7.53 | | m | (4) | Aromat |

1-(4'-tert.Butylphenyl)-4-(4'-methoxycarbonylphenoxy)-butandiol-1,3

a) Herstellung des Diastereomerengemisches

22.0 g (0.100 Mol) 1-(4'-tert.Butylphenyl)-3,4-epoxy-butanol-1, 15.2 g (0.100 Mol) 4-Hydroxybenzoesäuremethylester und 0.85 g (0.005 Mol) 4-Hydroxybenzoesäuremethylester Natriumsalz werden in 85 ml Dimethylformamid gelöst und 12 Stunden bei 100 °C gerührt. Nach Abkühlen der Reaktionsmischung gießt man in Eiswasser und extrahiert zweimal mit Ethylacetat. Die vereinigten organischen Phasen werden mit Wasser gewaschen und über $Na_2SO_4$ getrocknet. Nach Abziehen des Lösungsmittels i. Vak. bleibt das rohe Diastereomerengemisch als brauner, harziger Rückstand; Rohausbeute 36.8 g (99 %).

b) Trennung der Diastereomeren

Das Rohprodukt wird einer fraktionierenden Kristallisation aus Chlorbutan unterworfen. Die ersten Kristallfraktionen enthalten stark angereichert das Diastereomer A, das bei der Dünnschichtchromatographie an Kieselgel mit Chloroform/Ethylacetat 90/10 (V/V) als Fließmittel den höheren $R_f$- Wert aufweist. Es wird durch mehrfache Kristallisation aus Chlorbutan weiter gereinigt. Farblose Kristalle vom Schmp. 121-122 °C.

| $^1$H-NMR-Spektrum (100 MHz; $CDCl_3$): | | | |
|---|---|---|---|
| 1.31 | s | (9) | $(C\underline{H}_3)_3C$ |
| 1.90 - 2.13 | m | (2) | $CH\text{-}C\underline{H}_2\text{-}CH$ |
| 3.07 | d | (1) | O$\underline{H}$ (austauschbar) |
| 3.59 | d | (1) | O$\underline{H}$ (austauschbar) |
| 3.87 | s | (3) | $OC\underline{H}_3$ |
| 3.96 | d | (2) | $C\underline{H}_2O$ |
| 4.30 | m | (1) | $C\underline{H}OH$ |
| 5.01 | m | (1) | $ArC\underline{H}$ |
| 6.85 - 8.01 | m | (8) | Aromat |

Der Rückstand der Mutterlauge wird durch Chromatographie an Kieselgel mit Chloroform/Ethylacetat 90/10 (V/V) von Verunreinigungen befreit und das so vorgereinigte Diastereomerengemisch anschließend erneut einer fraktionierenden Kristallisation aus Chlorbutan unterworfen. Erste Kristallfraktionen stellen Mischungen dar, spätere enthalten stark angereichert das Diastereomer B mit kleinerem $R_f$-Wert. Sie werden durch Kristallisation aus Diisopropylether/Chlorbutan im Verhältnis 100/10, 90/20 und schließlich 75/35 weiter gereinigt. Farblose Kristalle vom Schmp. 103-106 °C.

| $^1$H-NMR-Spektrum (100 MHz; $CDCl_3$): | | | |
|---|---|---|---|
| 1.31 | s | (9) | $(C\underline{H}_3)_3C$ |
| 2.00 | m | (2) | $CHC\underline{H}_2CH$ |
| 2.87 | d | (1) | $O\underline{H}$ |
| 3.09 | d | (1) | $O\underline{H}$ |
| 3.87 | s | (3) | $OC\underline{H}_3$ |
| 4.00 | d | (2) | $OC\underline{H}_2$ |
| 4.29 | m | (1) | $C\underline{H}OH$ |
| 5.06 | m | (1) | $ArC\underline{H}$ |
| 6.85 - 8.01 | m | (8) | Aromat |

1-(4'-tert.Butylphenyl)-4-(4'-carboxyphenoxy)-butandiol-1,3: Diastereomer A

3.72 g (0.010 Mol) 1-(4'-tert.Butylphenyl)-4-(4'-methoxycarbonylphenoxy)-butandiol-1,3, Diastereomer A, werden mit 1.65 g (0.025 Mol) 85 % KOH in 30 ml Methanol und 5 ml Wasser 6 Stunden unter schwachem Rückfluß gekocht. Nach dem Abkühlen wird mit Wasser verdünnt, die Lösung mit konz. HCl angesäuert und das Produkt zweimal mit Ethylacetat extrahiert. Die vereinigten organischen Phasen werden mit Wasser gewaschen und über $Na_2SO_4$ getrocknet. Nach dem Abziehen des Lösungsmittels i. Vak. bleibt ein

kristalliner Rückstand, der aus Acetonitril/Methanol (7/1) unkristallisiert wird. Ausbeute 2.98 g (83 %); farblose Kristalle vom Schmp. 164-166 °C.
$C_{21}H_{26}O_5$ (358.4)
Molmasse 358 (massenspektroskopisch mittels Elektronenstoß-Ionisation (70 eV) bestimmt)
IR-Spektrum (KBr): ν (OH) 3600 - 2400 $cm^{-1}$ ν (C=O) 1680 $cm^{-1}$

| $^1$H-NMR-Spektrum (100 MHz; $CDCl_3/CD_3OD$): | | | |
|---|---|---|---|
| 1.31 | s | (9) | $(C\underline{H}_3)_3C$ |
| 1.90 - 2.10 | m | (2) | $CHC\underline{H}_2CH$ |
| 3.87 - 4.33 | m | (6) | $C\underline{H}C\underline{H}_2O$, 3 $O\underline{H}$ |
| 4.96 | dd | (1) | $ArC\underline{H}$ |
| 6.80 - 8.10 | m | (8) | Aromat |

1-(4'-tert.Butylphenyl)-4-(4'-carboxyphenoxy)-butandiol-1,3: Diastereomer B

In gleicher Weise wie vorstehend bei Diastereomer A beschrieben, wird 1-(4'-tert.Butylphenyl)-4-(4'-methoxycarbonylphenoxy)-butandiol-1,3, Diastereomer B, alkalisch verseift. Das glasige Rohprodukt kristallisiert aus Acetonitril. Ausbeute 91 %; farblose Kristalle vom Schmp. 140-143 °C.
$C_{21}H_{26}O_5$ (358.4)
Molmasse 358 (massenspektroskopisch)
IR-Spektrum (KBr): ν (OH) 3600 - 2400 $cm^{-1}$ ν (C=O) 1680 $cm^{-1}$

| $^1$H-NMR-Spektrum (100 MHz; $CDCl_3/CD_3OD$): | | | |
|---|---|---|---|
| 1.31 | s | (9) | $(C\underline{H}_3)_3C$ |
| 1.98 | m | (2) | $CHC\underline{H}_2CH$ |
| 3.80 - 4.40 | m | (6) | $C\underline{H}C\underline{H}_2O$, 3 $O\underline{H}$ |
| 4.99 | dd | (1) | $ArC\underline{H}$ |
| 6.80 - 8.10 | m | (8) | Aromat |

In entsprechender Weise lassen sich beispielsweise die folgenden erfindungsgemäßen Verbindungen herstellen:

(a) 1-(4'-Isopropylphenyl)-4-(4'-carboxyphenoxy)-butandiol-1,3;
(b) 1-[4'-(1,1-Dimethyl-2-hydroxyethyl)-phenyl]-4-(4'-carboxyphenoxy)-butandiol-1,3;
(c) 1-[4'-(1-Carboxy-1-methylethyl)-phenyl]-4-(4'-carboxyphenoxy)-butandiol-1,3;
(d) 1-(4'-tert.Butylphenyl)-4-[4'-(2,3-dihydroxypropoxycarbonyl)-phenoxy]-butandiol-1,3;
(e) 1-[4'-(1,1-Dimethyl-2-hydroxyethyl)-phenyl]-4-[4'-(2,3-dihydroxypropoxycarbonyl)-phenoxy]-butandiol-1,3.

Pharmakodynamische Effekte von 1-(4'-tert.Butylphenyl)-4-(4'-carboxyphenoxy)-butandiol-1,3: Diastereomer A und 1-(4'-tert.Butylphenyl)-4-(4'-carboxyphenoxy)-butandiol-1,3: Diastereomer B

1.) Bestimmung der hypolipidämischen Wirkung an Ratten

Die Überlegenheit der beanspruchten Verbindungen gegenüber dem bereits seit langem in die Therapie eingeführten Clofibrat lässt sich anhand der lipidsenkenden Wirkung eindeutig belegen.

Die lipidsenkende Wirkung der Testsubstanzen wurde an Gruppen von jeweils 10 normolipämischen, männlichen, 200 bis 220 g schweren Wistar Ratten geprüft.

Die Durchführung der Teste erfolgte nach einem 3-wöchigen Umtrainieren der Fressgewohnheiten der Tiere. Die kontrollierte Fütterung wurde täglich von 8:00 Uhr bis 10:00 Uhr durchgeführt. Die Applikation der Testsubstanzen erfolgte um 11:00 Uhr.

Die Testverbindungen wurden in einer wässrigen Lösung von 0.25% Agar und 0.84% Natriumchlorid aufgenommen und oral verabreicht. Nach dreimaliger Applikation über einen Zeitraum von drei Tagen wurde den Tieren Blut entnommen.

Die Bestimmung von Cholesterin und Triglyceriden im Serum wurde mit Hilfe des Zentrifugalanalysers "Cobas-Bio" von Hoffman-La Roche vorgenommen.

Methoden :

a.) Cholesterinbestimmung
CHOD-PAP-Methode, enzymatischer Farbtest nach J. Siedel et al. [J. Clin. Chem. Clin. Biochem. 19, 838 (1981)]
b.) Triglyceridbestimmung
Enzymatische Spaltung der Triglyceride mit Hilfe spezieller Lipasen mit nachfolgender Bestimmung des freigewordenen Glycerins nach T. O. Tiffany et al. [Clin. Chem. 20, 476 (1974)]

Tabelle 1

Prozentuelle Änderung der Gesamtcholesterin (TC)- und Triglycerid (TG)-Spiegel im Rattenserum nach oraler Applikation der Testsubstanzen

| Verbindungs-Nummer | Dosis (mg/kg) | % Änderung | |
|---|---|---|---|
| | | TC | TG |
| | | $\overline{X}$ ± SD | $\overline{X}$ ± SD |
| Vergleichssubs. Clofibrat | 100 | -14.5 ± 12.0 | -29.2 ± 19.4 |
| Diastereomer A | 100 | -39.1 ± 15.5 | -26.4 ± 13.1 |
| Diastereomer B | 100 | -66.9 ± 8.6 | -29.7 ± 15.5 |

2.) Bestimmung der Hemmung der Cholesterinsynthese

3 g Frisch präparierter Rattenleber wurden in 6 ml Eisgekühltem Puffer homogenisiert und bei 20.000 * g 20 Minuten lang zentrifugiert. Der Überstand wurde abgetrennt und für die nachfolgenden Bestimmungen verwendet.

Zu einer Mischung von 110 µl Überstand und 300 µl Inkubationslösung wurden 10 µl der methanolischen Testsubstanzlösung pipettiert und bei 37 °C 20 Minuten lang inkubiert. Anschliessend wurden 10 µl einer $^{14}C$-Azetat Lösung hinzupipettiert und weitere 30 Minuten inkubiert. Danach wurden zu der Mischung 500 µl äthanolischer KOH Lösung zugefügt, 60 Minuten lang bei 75 °C weiterinkubiert und anschliessend die Reaktion durch Abkühlen im Eisbad gestoppt. Um eine Ausbeutekorrektur für die nachfolgende Extraktion des entstandenen $^{14}C$-Cholesterins durchführen zu können, wurde $^{3}H$-Cholesterin ($\approx 10^{-3}$ µCi) dazupipettiert. Darüberhinanus wurden 500 µl einer äthanolischen Lösung kalten Cholesterins zugefügt. Das Cholesterin der Mischung wurde zweimal mit je 1 ml Petrol-Äther extrahiert, der Extrakt zur Trockene eingedampft und mit 0.6 ml einer Äthanol/Azeton Mischung (1/1) aufgenommen. Das gelöste Cholesterin wurde dann mittels 1.4 ml einer Digitoninlösung (=7 mg) ausgefällt. Nach 15 Stunden bei 0 °C wurde der Niederschlag abzentrifugiert, mit 0.5 ml Methanol/Eisessig (33/5) gelöst und mit 2 ml Methanol verdünnt. Die Radioaktivität des entstandenen Cholesterins wurde dann in 12 ml Scintillationsflüssigkeit (Dimilume) mittels eines Scintillations-Zählers bestimmt.

Verwendete Lösungen :

Puffer : 6.8 g $KH_2PO_4$, 0.6 g $MgCl_2$, 0.37 g EDTA, 1.83 g Nicotinamid, 51.35 g Saccharose und 0.44 ml Mercaptoäthanol wurden in destilliertem Wasser gelöst und bis 500 ml verdünnt ($P_H$ = 7.2)
Inkubationslösung : Jeweils einzeln aufgelöst wurden :
8.6 mg NAD mit 1.3 ml Puffer
10.2 mg NADP mit 1.3 ml Puffer
39.6 mg Glucose-6-Phosphat mit 0.65 ml Puffer
31.6 µl Glucose-6-Phosphat-Dehydrogenase (1000 U/0.9 ml) mit 0.285 Puffer
Diese Lösungen wurden erst unmittelbar vor der Verwendung gemischt.

$^{14}C$-Azetat :

Lösung 1 : 250 µCi $^{14}C$-Na-Azetat wurde mittels 1 ml dest. Wasser gelöst
Lösung 2 : 512.5 mg Na-Azetat wurde in 10 ml dest. Wasser gelöst

Vor dem Versuch wurden 0.1 ml der Lösung 1 und 0.4 ml der Lösung 2 gemischt und mit 2 ml dest. Wasser versetzt.

Ethanolische KOH : 10 g KOH wurden in 100 ml Äthanol gelöst.
Methode : R. E. Dugan et al. Archives Bioch. Biophys. 152 21, (1972)

Tabelle 2

| Prozentuelle Hemmung der Cholesterinbiosynthese im Rattenleberhomogenat durch die Testsubstanzen | | |
|---|---|---|
| Diastereomer | Konzentration (Mol/l) | % Kontrolle |
| A | $3*10^{-7}$ | 122 |
| A | $1*10^{-6}$ | 103 |
| A | $3*10^{-6}$ | 97 |
| A | $1*10^{-5}$ | 36 |
| A | $3*10^{-5}$ | 14 |
| A | $1*10^{-4}$ | 9 |
| A | $3*10^{-4}$ | 2 |
| B | $1*10^{-5}$ | 42 |

## Patentansprüche

**1.** p-Oxibenzoesäurederivate der allgemeinen Formel (1)

(1) 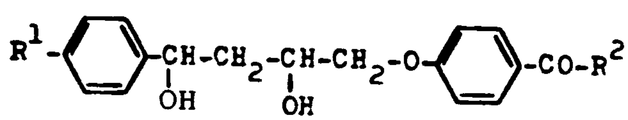

in der bedeuten:

$R^1$ =

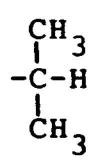 , 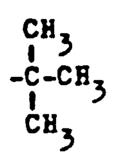 , 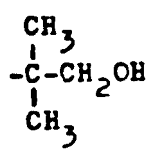 oder 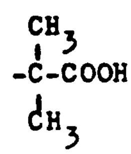

und

$R^2$ = -OH ,

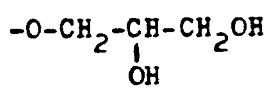 oder 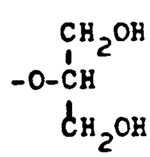

ihre Diastereomere und Enantiomere in reiner Form oder als Gemisch stereoisomerer Formen sowie

ihre physiologisch verträglichen Salze.

2. 1-(4'-tert.Butylphenyl)-4-(4'-carboxyphenoxy)-butandiol-1,3 in Form seiner beiden voneinander getrennten Diastereomeren, in Form eines Diastereomerengemisches oder als reine Enantiomere.

3. Verfahren zur Herstellung von p-Oxibenzoesäurederivaten nach Anspruch 1,
dadurch **gekennzeichnet,** daß man in an sich bekannter Weise Epoxide der allgemeinen Formel (2)

(2) 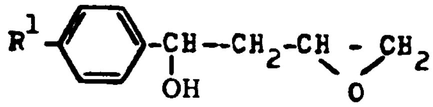

in der
$R^1$ =

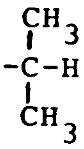 , 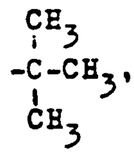 , 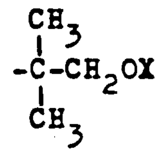 oder 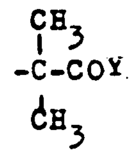

bedeutet und sowohl X als auch Y eine geeignete Schutzgruppe darstellt, mit den entsprechenden p-Oxibenzoesäureestern umsetzt, gegebenenfalls die erhaltenen Ester verseift, gegebenenfalls vorhandene Schutzgruppen abspaltet sowie gegebenenfalls das erhaltene Diastereomerengemisch trennt, wobei die Trennung vor oder nach Abspaltung der Schutzgruppe erfolgen kann.

4. Verfahren nach Anspruch 3,
dadurch **gekennzeichnet,** daß man die Diastereomeren durch fraktionierte Kristallisation trennt.

5. Verfahren zur Herstellung von 1-(4'-tert.Butylphenyl)-4-(4'-carboxyphenoxy)-butandiol-1,3,
dadurch **gekennzeichnet,** daß man (1) 4-tert.Butylbenzaldehyd mit einem Allylmagnesiumhalogenid zu 1-(4'-tert.Butylphenyl)-buten-3-ol-1 umsetzt, daß man (2) das erhaltene 1-(4'-tert.Butylphenyl)-buten-3-ol-1 zu 1-(4'-tert.-Butylphenyl)-3,4-epoxy-butanol-1 oxidiert, daß man (3) das erhaltene 1-(4'-tert.Butylphenyl)-3,4-epoxy-butanol-1 mit 4-Hydroxybenzoesäuremethylester zu einem 1-(4'-tert.Butylphenyl)-4-(4'-methoxycarbonylphenoxy)butandiol-1,3-Diastereomerengemisch umsetzt, daß man (4) die Diastereomeren voneinander trennt und daß man (5) die getrennten Ester alkalisch verseift.

6. Verwendung von p-Oxibenzoesäurederivaten nach Anspruch 1, zur Herstellung eines Arzneimittels mit hypolipämischer Wirkung.

## Claims

1. p-Oxybenzoic acid derivatives of the general formula (1)

(1) 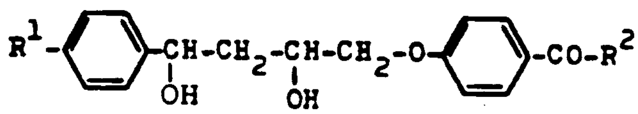

wherein represent:
$R^1$ =

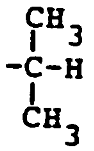 , 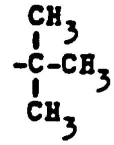 , 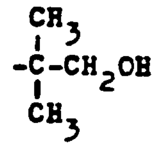 or 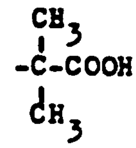

and

$R^2$ = -OH ,

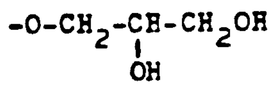 or 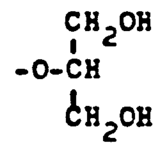

the diastereomers and enantiomers thereof in pure form or as a mixture of stereoisomeric forms as well as the physiologically acceptable salts thereof.

**2.** 1-(4'-tertiary Butylphenyl)-4-(4'-carboxyphenoxy)-butanediol-1,3 in form of its diastereomers provided separated from each other, in form of a mixture of the diasteromers or as pure enantiomers.

**3.** A process for the preparation of p-oxybenzoic acid derivatives according to claim 1, characterized in that epoxides of the general formula (2)

(2) 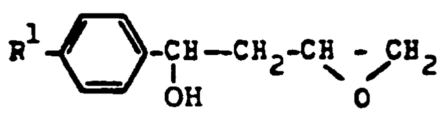

wherein

$R^1$ =

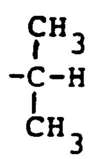 , 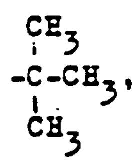 , 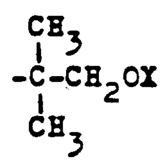 or 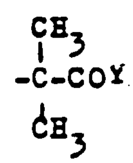

and X and Y, both, are representing a suitable protecting group, are reacted with the corresponding p-oxybenzoic acid esters in a known manner, the obtained esters are eventually hydrolyzed, eventually present protecting groups are cleaved off and the obtained mixture of diastereomers is eventually separated, wherein the separation may be performed prior to or after cleaving off the protecting group.

**4.** A process according to claim 3, characterized in that the diastereomers are separated by fractionated crystallization.

**5.** A process for the preparation of 1-(4'-tertiary butylphenyl)4-(4'-carboxyphenoxy)-butanediol-1,3, characterized in that

(1) 4-tertiary butylbenzaldehyde is reacted with an allyl magnesium halide to obtain 1-(4'-tertiary butylphenyl)-butene-3-ol-1,

(2) the obtained 1-(4'-tertiary butylphenyl)-butene-3-ol-1 is oxidized to obtain 1-(4'-tertiary butylphenyl-3,4-epoxy-butanol-1,

(3) the obtained 1-(4'-tertiary butylphenyl-3,4-epoxy-butanol-1 is reacted with 4-hydroxybenzoic acid methyl ester to obtain a mixture of diastereomeric 1-(4'-tertiary butylphenyl)-4-(4'-methoxycarbonylphenoxy)butanediol-1,3,

(4) said diastereomers are separated from each other and
(5) the separated esters are subjected to alkaline hydrolysis.

**6.** Use of the p-oxybenzoic acid derivatives according to claim 1 for the preparation of a medicament having hypolipaemic activity.

**Revendications**

**1.** Dérivés de l'acide p-oxybenzoïque de formule générale (1)

(1) 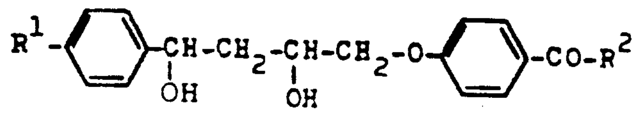

dans laquelle :
$R^1$ représente un groupe

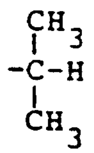 , 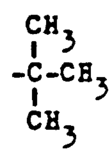 , 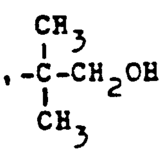 ou 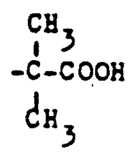

et
$R^2$ représente un groupe -OH,

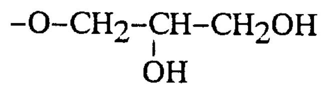 ou 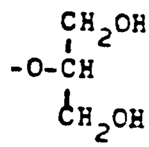

leurs diastéréomères et énantiomères sous forme pure ou sous des formes de mélanges des stéréoisomères, ainsi que leurs sels physiologiquement tolérables.

**2.** Le 1-(4'-tert.butylphényl)-4-(4'-carboxyphénoxy)-butanediol-1,3 sous forme de ses diastéréomères séparés l'un de l'autre, sous forme d'un mélange de diastéréomères ou sous forme des énantiomères purs.

**3.** Procédé de préparation de dérivés d'acides p-oxybenzoïques selon la revendication 1, procédé caractérisé en ce qu'on fait réagir, de façon connue en soi, des époxydes de formule générale (2)

(2) 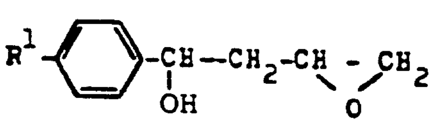

dans laquelle $R^1$ représente un groupe

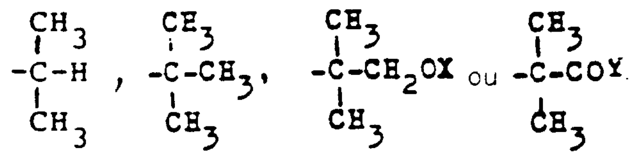

, , ou

et X aussi bien que Y représentent un groupe protecteur convenable, avec les esters correspondants d'acides p-oxybenzoïques, on saponifie éventuellement les esters obtenus, on scinde éventuellement les groupes protecteurs présents et l'on sépare éventuellement le mélange des diastéréomères ainsi obtenus, la séparation pouvant avoir lieu avant ou après scission des groupes protecteurs.

**4.** Procédé selon la revendication 3, caractérisé en ce qu'on sépare par cristallisation fractionnée les diastéréomères.

**5.** Procédé de préparation du 1-(4'-tert.-butylphényl)-4-(4'-carboxyphénoxy)-butanediol-1,3, caractérisé en ce que (1) on fait réagir le 4-tert.-butylbenzaldéhyde avec un halogénure d'allylmagnésium ce qui donne le 1-(4'-tert.-butylphényl)-butène-3-ol-1, (2) on oxyde le 1-(4'-tert.butylphényl)-butène-3-ol-1 ainsi obtenu pour obtenir le 1-(4'-tert.-butylphényl)-3,4-époxy-butanol-1, (3) on fait réagir le 1-(4'-tert.butylphényl)-3,4-époxy-butanol-1 ainsi obtenu avec l'ester méthylique de l'acide 4-hydroxybenzoïque, ce qui donne un mélange des diastéréomères du 1-(4'-tert.-butylphényl)-4-(4'-méthoxycarbonylphénoxy)-butanediol-1,3, (4) on sépare l'un de l'autre les diastéréomères et (5) on saponifie par voie alcaline les esters ainsi séparés.

**6.** Utilisation des dérivés d'acide p-oxybenzoïque selon la revendication 1 pour préparer un médicament à action hypolipémiante.